# EUROPÄISCHE PATENTSCHRIFT

(11) **EP 0 769 002 B1**
(45) Veröffentlichungstag und Bekanntmachung des Hinweises auf die Patenterteilung: **21.10.1998**
(21) Anmeldenummer: 95924972.3
(22) Anmeldetag: 05.07.1995
(51) Int. Cl.: C07C 231/08, C07C 233/20

(54) **VERFAHREN ZUR HERSTELLUNG VON KRISTALLISIERTEM N-METHYLOL-(METH)ACRYLAMID**
METHOD OF PRODUCING CRYSTALLISED N-METHYLOL-(METH)ACRYLAMIDE
PROCEDE DE FABRICATION DE N-METHYLOL-(METH)ACRYLAMIDE CRISTALLISE

(30) Priorität: 06.07.1994 DE 4423740
(43) Veröffentlichungstag der Anmeldung: 23.04.1997
(73) Patentinhaber: BASF AKTIENGESELLSCHAFT, 67056 Ludwigshafen (DE)
(72) Erfinder: BOTT, Kaspar, D-68165 Mannheim (DE); DOMSCHKE, Thomas, D-67346 Speyer (DE); BOHN, Michael, D-67069 Ludwigshafen (DE)
(74) Vertreter: Isenbruck, Günter, Dr.
(86) Internationale Anmeldenummer: EP9502594
(87) Internationale Veröffentlichungsnummer: WO9601251

(56) Entgegenhaltungen:
- US-A- 2 864 861
- US-A- 2 864 862

## Beschreibung

Die Erfindung betrifft ein Verfahren zur Herstellung von kristallisiertem N-Methylol-(meth)acrylamid, aus Paraformaldehyd und (Meth)acrylamid in Gegenwart eines geeigneten Katalysators.

N-Methylol-(meth)acrylamid kommt als Monomer-Komponente mit Vernetzereigenschaften in einer Reihe von Polymerisaten, überwiegend in Polymerdispersionen, zum Einsatz, die als Klebstoffe, Anstrichmittel, als Bindemittel für Farben und Fasern und als Material für die Textil- oder Papierbeschichtung Anwendung finden.

Verfahren zur Herstellung von kristallisiertem N-Methylol-acrylamid sind bekannt. So ist in der US-Patentschrift 3,064,050 ein derartiges Verfahren beschrieben, bei dem Acrylamid und Paraformaldehyd in hochkonzentrierter wäßriger Lösung mit einem basischen Katalysator zur Umsetzung gebracht werden. Die Nachteile dieses Verfahrens sind a) die relativ niedrigen Produktausbeuten von etwa 50%, die man bei einem Arbeitsgang erhält, b) die deshalb erforderliche Rückführung der Mutterlaugen und c) die Trocknung des durch Abfiltrieren gewonnenen Produkts.

Aus der US-Patentschrift 2,864,861 ist zwar ein Verfahren zur Gewinnung von N-Methylol-acrylamid bekannt, das auf jeglichen Einsatz von Lösungsmitteln verzichtet. Entsprechend diesem bekannten Verfahren wird in einem Rührkessel aus den festen Reaktionskomponenten Paraformaldehyd und Acrylamid mit einer katalytischen Menge von Triethylamin eine Produktschmelze erzeugt, die man nach Beendigung der Reaktion durch Kühlung zu festem N-Methylol-acrylamid erstarren läßt. Bei einer Übertragung dieses Verfahrens auf eine großtechnische Anlage wäre es nicht möglich, folgendes zu erreichen:
a) die Reaktionskomponenten gleichmäßig zu vermischen;
b) die Reaktionswärme zügig abzuführen, um einen Wärmestau und damit die Polymerisationsgefahr zu vermeiden;
c) ein N-Methylol-acrylamid gleichbleibender Qualität zu erzeugen und
d) das N-Methylol-acrylamid in rieselfähiger Form aus dem Rührkessel auszutragen.

Der Erfindung liegt daher die Aufgabe zugrunde, ein lösungsmittelfreies Verfahren zur Herstellung von N-Methylol-(meth)acrylamid zu entwickeln, bei dem auch im großtechnischen Maßstab die oben genannten Merkmale erreichbar sind.

Diese Aufgabe wird erfindungsgemäß durch die in den Ansprüchen definierten Verfahren gelöst. Insbesondere werden (Meth)acrylamid und Paraformaldehyd mit einem geeigneten Katalysator in einem Verfahrensschritt zu dem kristallisierten N-Methylol-(meth)acrylamid umgesetzt indem die Umsetzung in einem Reaktor, vorzugsweise in einer selbstreinigenden Schneckenapparatur, durchgeführt wird. Der Reaktor ist dabei so ausgelegt, daß das Reaktionsgemisch signifikanten Scherbelastungen, wie bei Schneckenextrudern oder bei Scheibenmischern, ausgesetzt werden. Diese Apparaturen sind an sich bekannt und damit ist auch der Grad bzw. die Intensität des mechanischen Mischens für Reaktion und Kristallisation definiert. Vorzugsweise werden Reaktoren eingesetzt, die eine Scherbelastung auf das Reaktionsgemisch übertragen, die gemessen am Leistungsverbrauch pro Füllvolumeneinheit größen ordnungsgemäßig gleich derjenigen der in den Beispielen genannten, kommerziellen Geräte bei gleichem Füllmaterial ist.

Unter "(Meth)acrylamid" soll im folgenden Acrylamid und/oder Methacrylamid verstanden werden. Als Katalysatoren sind tertiäre Amine oder Alkalicarbonate, besonders aber Kaliumcarbonat geeignet. Dieses Verfahren, das sowohl in kontinuierlicher als auch in diskontinuierlicher Fahrweise betrieben werden kann, liefert ein rieselfähiges Produkt mit geringen Qualitätsschwankungen. Die Funktionsweise des zur Ausübung des vorliegenden Verfahrens eingesetzten Discotherm B soll im folgenden näher erläutert werden:

Zur Herstellung von N-Methylol-(meth)acrylamid werden vorzugsweise Reaktoren mit hohem Selbstreinigungsgrad verwendet. Ein solcher Reaktor ist beispielsweise ein Discotherm B von der Fa. List AG, 4422 Arisdorf, Schweiz, in dem sich die erforderliche Verweilzeit realisieren läßt.

Der Discotherm B (DTB) ist eine ansatzweise (batch-wise) oder kontinuierlich arbeitende Knetmaschine. Sie besteht aus einem horizontalen zylindrischen Gehäuse mit einer konzentrischen Rührwelle, auf der in Ebenen senkrecht zur Achse Scheibenelemente und am äußeren Umfang Knet-Mischbarren aufgesetzt sind. Im Gehäuse sind statische Gegenhaken befestigt, die die Welle und die Scheibenelemente reinigen. Der Selbstreinigungsgrad der Maschine beträgt 90%. Die verbleibenden 10% werden durch Produktbewegungen abgereinigt.

Der axiale Transport wird durch die Anordnung der Scheibenelemente mit Knet-Mischbarren und durch die Form der Gegenhaken bewirkt. Die Axialvermischung ist gering, so daß ein enges Verweilzeitspektrum realisiert werden kann.

Gehäuse, Welle und Scheibenelemente sind heiz- und kühlbar. Der Füllgrad (vorzugsweise 60 - 80%) kann über die Höhe eines verstellbaren Überlaufwehres am Maschinenaustrag eingestellt werden.

### Beispiel 1

### Diskontinuierliche Herstellung von N-Methylol-acrylamid

Die Einsatzstoffe Acrylamid, Paraformaldehyd und Kaliumcarbonat liegen in Form von rieselfähigen Pulvern vor. In einem Discotherm B, Typenbezeichnung DTB-40-Batch (freies Volumen ca. 60 l), werden 13,78 kg Acrylamid, 6,12 kg Paraformaldehyd und 0,1 kg Kaliumcarbonat eingefüllt und bei einer Drehzahl von 50 min⁻¹ vermischt. Der Reaktor wird auf 50 °C aufgeheizt. Nach ca. 30 min ist die Reaktion abgeschlossen und das N-Methylol-acrylamid liegt in Form einer klaren Schmelze vor. Der Reaktor wird nun auf ca. 20 °C abgekühlt, so daß die N-Methylol-acrylamid-Schmelze zu kristallisieren beginnt. Nach einer Abkühldauer von 45 min ist das N-Methylol-acrylamid vollständig auskristallisiert und wird in Form eines feinkörnigen Pulvers aus dem Reaktor ausgetragen. Das erhaltene Produkt hat einen Schmelzpunkt von 53 - 57 °C.

### Beispiel 2

### Kontinuierliche Herstellung von N-Methylol-acrylamid

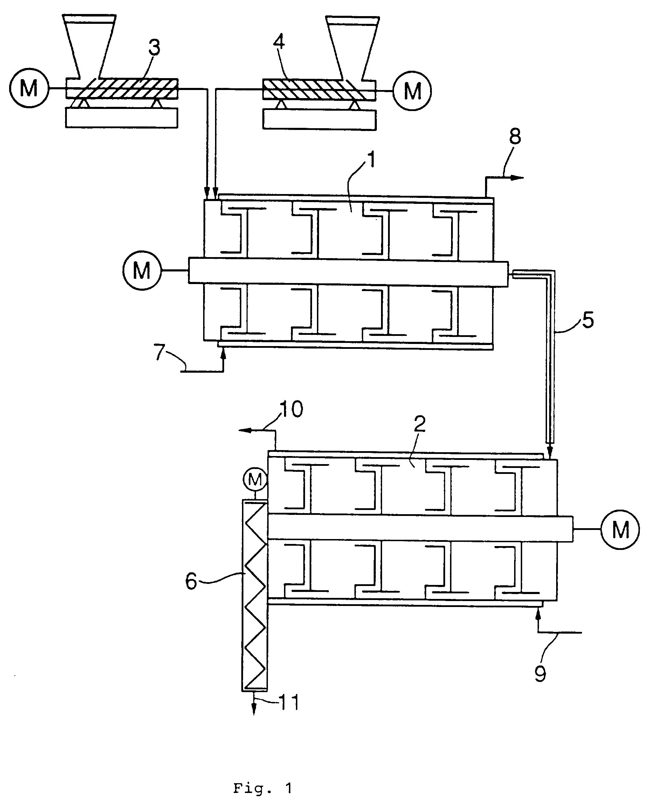

Der Aufbau der Apparatur ist in der Figur dargestellt. Die verschiedenen Antriebsaggregate sind dort allgemein mit "M" bezeichnet. Dem Reaktor 1 vom Typ Discotherm B (DTB 40-Conti) werden mit Hilfe von zwei Differentialdosierwaagen 3 und 4 vom Typ K-Tron Soder 20,67 kg/h Acrylamid, 9,18 kg/h Paraformaldehyd und 0,15 kg/h Kaliumcarbonat kontinuierlich zudosiert (Gesamtdurchsatz 30 kg/h). Paraformaldehyd und Kaliumcarbonat werden als Mischung zudosiert. Der Reaktor 1 wird mit durch die Leitung 7 zuströmenden Warmwasser (50 °C), das durch Leitung 8 wieder abfließt, beheizt. Die Wellendrehzahl beträgt 50 min⁻¹. Bei einem Füllgrad von ca. 60% und einer Verweilzeit > 35 min reagieren die Feststoffe zu einer klaren Schmelze, welche kontinuierlich durch eine mit Warmwasser auf 50 °C beheizte Rohrleitung 5 unter Schwerkrafteinfluß in den zweiten Reaktor 2 fließt. Der zweite Reaktor 2 ist ebenfalls ein DTB 40-Conti, der mit durch die Leitung 9 zuströmendem - und durch Leitung 10 abströmenden Kühlwasser auf 20 °C temperiert wird, so daß die Schmelze kristallisiert. Bei einer Wellendrehzahl von 25 min⁻¹, einem Füllgrad von 60% und einer Verweilzeit > 35 min erhält man am Ende des Reaktors einen feinkörnigen Feststoff, der mit einer vertikalen Förderschnecke 6 kontinuierlich über die Leitung 11 ausgetragen wird.

## Patentansprüche

1. Verfahren zur Herstellung von kristallisiertem N-Methylol-(meth)acrylamid aus (Meth)acrylamid und Paraformaldehyd, **dadurch gekennzeichnet, daß** die Umsetzung der festen Reaktionskomponenten zu einer Produktschmelze sowie deren anschließende Kristallisation in einem Reaktor unter mechanischer Scherbelastung des Reaktionsgemisches, vorzugsweise in einem selbstreinigenden Schneckenreaktor oder einem Scheibenreaktor, durchgeführt wird.

2. Verfahren nach Anspruch 1, dadurch gekennzeichnet, daß die Umsetzung der Reaktionskomponenten und die Kristallisation der Produktschmelze ohne zugefügtes Lösungsmittel durchgeführt wird.

3. Verfahren nach Anspruch 1 oder 2, dadurch gekennzeichnet, daß ein Katalysator für die Umsetzung der Reaktionskomponenten, insbesondere ein Alkalicarbonat oder ein Trialkylamin, verwendet wird.

4. Verfahren nach einem der vorhergehenden Ansprüche, dadurch gekennzeichnet, daß als selbstreinigender Schneckenreaktor ein gleichsinnig drehender Doppelwellenextruder verwendet wird.

5. Verfahren nach einem der vorhergehenden Ansprüche, dadurch gekennzeichnet, daß das Verfahren in einem Reaktor durchgeführt wird, der aus zwei Abschnitten besteht, wobei in dem ersten Abschnitt, der die Scherbelastung des Reaktionsgemisches gestattet, im wesentlichen die chemische Umsetzung erfolgt, während die Kristallisation in dem zweiten Abschnitt erfolgt.

6. Verfahren nach Anspruch 5, dadurch gekennzeichnet, daß der erste und der zweite Abschnitt des Reaktors unabhängig voneinander regelbare Scherbelastungseinrichtungen aufweisen.

7. Verfahren nach einem der vorhergehenden Ansprüche, dadurch gekennzeichnet, daß das Verfahren kontinuierlich durchgeführt wird.

8. Verfahren nach Anspruch 7, dadurch gekennzeichnet, daß die Materialien durch den Reaktor mit einer engen Verteilung der Verweilzeit für die eingesetzten Materialien geführt werden.

## Claims

1. A process for the preparation of crystalline N-methylol(meth)acrylamide from (meth)acrylamide and paraformaldehyde, wherein the reaction of the solid reactants to give a product melt and the subsequent crystallization thereof in a reactor while subjecting the reaction mixture to mechanical shear load is preferably carried out in a self-purging screw reactor or a disk reactor.

2. A process as claimed in claim 1, wherein the reaction of the reactants and the crystallization of the product melt are carried out without added solvent.

3. A process as claimed in claim 1 or 2, wherein a catalyst, in particular an alkali metal carbonate or a trialkylamine, is used for the reaction of the reactants.

4. A process as claimed in any of the preceding claims, wherein the self-purging screw reactor used is a twin-screw extruder having screws rotating in the same direction.

5. A process as claimed in any of the preceding claims, which is carried out in a reactor which consists of two sections, essentially the chemical reaction taking place in the first section, which allows the reaction mixture to be exposed to a shear load, while the crystallization takes place in the second section.

6. A process as claimed in claim 5, wherein the first section and the second section of the reactor have shear load means which can be regulated independently of one another.

7. A process as claimed in any of the preceding claims, which is carried out by a continuous method.

8. A process as claimed in claim 7, wherein the materials are passed through the reactor with a narrow distribution of the residence time for the materials used.

## Revendications

1. Procédé de fabrication de N-méthylol-(méth)acrylamide cristallisé à partir de (méth)acrylamide et de paraformaldéhyde, caractérisé par le fait que la conversion des composants solides de réaction en un produit fondu, ainsi que leur cristallisation subséquente, sont effectuées dans un réacteur, avec exposition à un cisaillement d'origine mécanique du mélange de réaction, de préférence dans un réacteur à vis ou un réacteur à disque, auto-nettoyeur.

2. Procédé selon la revendication 1, caractérisé par le fait que la conversion des composants de réaction et la cristallisation du produit en fusion sont effectuées sans addition de solvant.

3. Procédé selon la revendication 1 ou 2, caractérisé par le fait qu'un catalyseur destiné à la conversion des composants de réaction, en particulier un carbonate alcalin ou trialkylamine, est utilisé.

4. Procédé selon l'une des revendications précédentes, caractérisé par le fait qu'on utilise comme réacteur à vis auto-nettoyeur une extrudeuse à arbre double, les arbres tournant dans le même sens.

5. Procédé selon l'une des revendications précédentes, caractérisé par le fait que le procédé est mis en oeuvre dans un réacteur constitué de deux tronçons, dans le premier tronçon, qui permet l'exposition à un cisaillement du mélange de réaction, étant effectuée essentiellement la conversion chimique, tandis que la cristallisation est effectuée dans le deuxième tronçon.

6. Procédé selon la revendication 5, caractérisé par le fait que le premier et le deuxième tronçon du réacteur présentent des dispositifs d'exposition au cisaillement, réglables indépendamment l'un de l'autre.

7. Procédé selon l'une des revendications précédentes, caractérisé par le fait que le procédé est mis en oeuvre de façon continue.

8. Procédé selon la revendication 7, caractérisé par le fait que les matériaux passés dans le réacteur sont conduits avec une dispersion étroite du temps de séjour pour les matériaux mis en oeuvre.
